# EUROPEAN PATENT APPLICATION

(11) **EP 2 725 097 A1**
(43) Date of publication of application: **30.04.2014**
(21) Application number: 12803695.1
(22) Date of filing: 25.06.2012
(51) Int. Cl.: C12M 3/00

(54) **CELL CULTURE DEVICE AND CELL CULTURE METHOD**

(30) Priority: 27.06.2011 JP 2011141385
(71) Applicant: Hitachi, Ltd., Tokyo 100-8280 (JP)
(72) Inventor: NAKAJIMA Ryota, Tokyo 100-8220 (JP); MATSUOKA Shizu, Tokyo 100-8220 (JP); KOBAYASHI Toyoshige, Tokyo 100-8220 (JP)
(74) Representative: Strehl Schübel-Hopf & Partner
(86) International application number: PCT/JP2012/066103
(87) International publication number: WO 2013/002158

(57) **Abstract**

Provided is a cell culture device having a culture area for culturing the stem or progenitor cells, from which tissues are formed. The cell culture device includes an oxygen adjusting part that adjusts the oxygen supply in the culture area, a controller that controls the oxygen adjusting part, and another controller that controls a first oxygen supply during a first period of the period, in which the stem cells and the progenitor cells differentiate; and a second oxygen supply greater in amount than the first supply during a second period, during which the stem cells and the progenitor cells differentiate, out of the whole culture period, and that controls the oxygen adjusting part based on the growth progress of the stem cells and the progenitor cells through self-replication to change the first oxygen supply to the second oxygen supply.

## Description

### Technical Field

The present invention relates to a cell culture device and a cell culture method.

### Background Art

Recently, 3-diensional (3D) tissues regenerated *in vitro,* which have properties similar to those of living bodies, have come into the picture from the standpoints of improving therapeutic effects in regeneration medicine, which uses cells to treat diseases, and increasing the efficiency of development of new drugs and cosmetics using alternative animal studies and human-derived cells. To produce regenerated tissues *in vitro,* cells need to be cultured by a skilled technician. However, this manual method for cell culture puts a burden on the technician and is high in cost. Because of its manual process, constant level of quality cannot be achieved. To solve this problem, a method for automatically culturing cells is being sought. Taking an example of producing stratified epithelial cell sheets, it takes about two weeks for corneal epithelial sheets and oral mucosal epithelial sheets and three weeks for epidermal sheets; it is expected that cells will be cultured in a shorter period. A method for automating a cell culture process is disclosed in Patent Literature 1. A method for facilitating the growth of epithelial stem cells by culturing the stem cells at an oxygen (O₂) level lower than about 20% in the normal cell culture environment is disclosed in Nonpatent Literature 1.

### Citation List

### Patent Literature

Patent Literature 1: Japanese Unexamined Patent Application Publication No. 2006-149237

### Nonpatent Literature

Nonpatent Literature 1: H. Miyashita et al.: Hypoxia enhances the expansion of human limbal epithelial progenitor cells in vitro: Investigative Ophthalmology & Visual Science, 48, 3586-3593, 2007

### Summary of Invention

### Technical Problem

However, an apparatus for automatically culturing cells rapidly by means of changes in oxygen supply is not disclosed in Patent Literature 1; thereby, a problem of taking long time to supply the cultured tissues remains unsolved. In Nonpatent Literature 1, no solution has been provided to a problem with the whole culture period, namely how to reduce the culture period including a period, during which 3D tissues generally incapable of being cultured at a low oxygen level are produced from the cells cultured at the low oxygen level.

In light of these problems, an object of the present invention is to provide a cell culture device and a method, which enable the culture period to be reduced.

### Solution to Problem

The cell culture device of the present invention is characterized in that it has a culture area for culturing stem cells or progenitor cells used to produce tissues, and includes an oxygen adjusting part that adjusts an oxygen level in the culture area, a controller that controls the oxygen adjusting part, and another controller that controls a first oxygen supply in a first period of the period, during which the stem cells and the progenitor cells self-replicate, and a second oxygen supply, greater in amount than the first oxygen supply, in a second period including a period, during which the stem cells or the progenitor cells differentiate, out of the whole cell culture period, and that controls the oxygen adjusting part so that it changes the first oxygen supply to the second oxygen supply based on the growth progress of the stem cells or the progenitor cells through self-replication.

### Advantageous Effects of Invention

According to the cell culture device and the method for cell culture of the present invention, a tissue may be produced in a short time period.

### Brief Description of Drawings

Fig. 1 is a view showing the images of epithelial cells under culture, taken by a phase-contrast microscope.
Fig. 2 is a diagram showing the counts of corneal epithelial cells contained in cell sheets on day 12 and on day 14 of culture.
Fig. 3 is a diagram showing cell growth curves.
Fig. 4 is a view showing the hematoxylin-eosin (HE) stained images in the sections of corneal epithelial cells.
Fig. 5 is a view showing the immunostained images in the corneal epithelial cell sheets.
Figs. 6A and 6B are views showing the rate of the stem/progenitor cells with maker p63 positive in the corneal epithelial cell sheets.
Figs. 7A and 7B are views showing the rate of colony formation of the stem/progenitor cells in the corneal epithelial cell sheets.
Fig. 8 is a scatter plot of about 30,000 expressed genes obtained by an exhaustive analysis of gene expression.
Fig. 9 is a view showing the confluent and cobblestone states of the cells.
Fig. 10 is a diagram illustrating the apparatus configuration when the oxygen level is changed across the whole culture tank.
Fig. 11 is a diagram illustrating the apparatus configuration with an optical coherence tomography added to the configuration shown in Fig. 10.
Fig. 12 is a diagram illustrating the apparatus configuration with an electric resistance measuring apparatus added to the configuration shown in Fig. 10.
Fig. 13 is a diagram illustrating the apparatus configuration with the electric coherence tomography and a transepithelial electric resistance measuring apparatus added to the configuration shown in Fig. 10.
Fig. 14 is a diagram illustrating the apparatus configuration when the oxygen level is directly changed in a culture container.
Fig. 15 is a diagram illustrating the apparatus configuration with the electric resistance measuring apparatus added to the configuration shown in Fig. 14.
Fig. 16 is a diagram illustrating the apparatus configuration with the electric coherence tomography and the transepithelial electric resistance measuring apparatus added to the configuration shown in Fig. 14.
Fig. 17 is a diagram illustrating the apparatus configuration with the electric coherence tomography and the transepithelial electric resistance measuring apparatus added to the configuration shown in Fig. 14.
Fig. 18 is a diagram illustrating the apparatus configuration, when the oxygen level is directly changed in a culture container, for which a gas-permeable membrane may be changed.
Fig. 19 is a diagram illustrating the apparatus configuration with the electric resistance measuring apparatus added to the configuration shown in Fig. 18.
Fig. 20 is a diagram illustrating the apparatus configuration with the electric coherence tomography and the transepithelial electric resistance measuring apparatus added to the configuration shown in Fig. 18.
Fig. 21 is a diagram illustrating the apparatus configuration with the electric coherence tomography and the electric resistance measuring apparatus added to the configuration shown in Fig. 18.
Fig. 22 is a view showing the culture container, for which gas-permeable membrane may be changed.
Fig. 23 is a schematic diagram showing the time points when the cells exhibit its confluent state, cobblestone state, or tightly-associated state.
Fig. 24 is a diagram illustrating the relationship between the count of culture period (days) and the average size of the cells.
Figs. 25A and 25 B are views representing the average size of the cells in the confluent or cobblestone state.
Fig. 26 is a view showing a cell species, to which this example of the present invention is applicable.

### Description of Embodiments

Embodiments of the present invention are explained taking examples. It should be noted that these embodiments and examples are only illustrative examples to implement the present invention and do not limit the technical scope of the present invention. Moreover, the same reference signs are assigned to the common parts in individual figures.

### Examples

### Example 1

With respect to this example, the principle and method of reducing the period, during which regenerated tissues are produced under the control of oxygen level, are explained. Hereinafter, using rabbit corneal epithelial cell sheets as a model, the principle and method are explained. The cell species is not limited to rabbits, may include the cells derived from mammalians such as mice, rats, canines, and humans. Moreover, the cell species is not limited to corneal epithelial cells and may include other epithelial cells derived from the skin and oral mucosa, as well as the stem cells or progenitor cells derived from the connective tissue, muscle tissue, nervous tissue, and sensory organ, which are used to produce tissues.

The culture period, during which the cell species used to produce tissues are cultured, may be divided into a self-replication period, during which the stem cells or progenitor cells self-replicate and a differentiation period, during which the cells, after covering over the culture surface of a medium to the given extent, differentiate. Hereinafter, taking an example of corneal epithelial cell sheets, the principle of reducing the culture period is described. Note that the cell differentiation in the corneal epithelial cell sheet is specifically called stratification, namely formation of a laminated structure by the cells; accordingly, the differentiation period is referred to as the stratification period in the following explanation.

First of all, taking the illustrative examples described below, the principle of the method of the present invention is explained, which enables the cells to be cultured at a low oxygen level during the self-replication period and cultured at the normal oxygen level during the stratification period to produce the stratified epithelial cell sheets in a shorter time period than those of conventional methods for culturing the cells at the normal oxygen level.

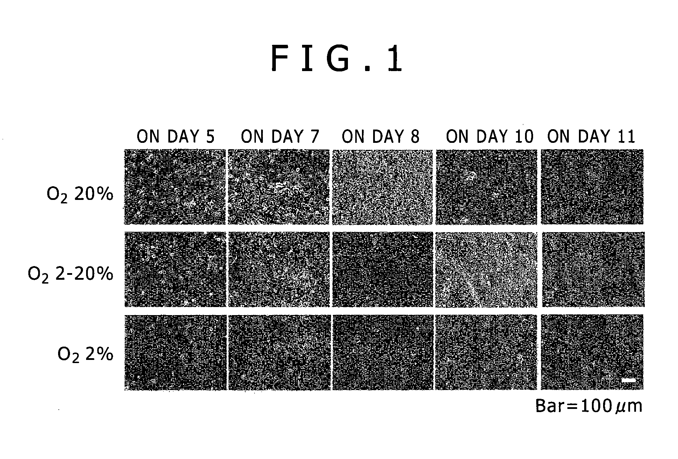

The scenario, in which the cells are cultured at 2% of O₂ level during the self-replication period and cultured at 20% of O₂ level (normal level) during the stratification period, is assumed to be an example 1; the scenario, in which he cells are cultured at 2% of O₂ level during both the periods, is assumed to be a comparative example 1; and the scenario, in which the cells are cultured at 20% of O₂ level during both the periods, is assumed to be a comparative example 2. Fig. 1 shows the images of the cells taken by the phase-contrast microscope in the illustrative example 1, and the comparative examples 1 and 2. In the comparative example 1, the cells covered densely across the whole culture on day 10 of culture (hereinafter, referred to as a confluent state), and then they were condensed by growth through self-replication into the smaller cells tightly laid (hereinafter, referred to as a cobblestone state); while, the cells cultured at 2% of O₂ level in the illustrative example 1 and the comparative example 2 exhibited the cobblestone state on around 8 day of culture. This suggests that the period required to reach the cobblestone state may be reduced by about two days compared the comparative example 1.

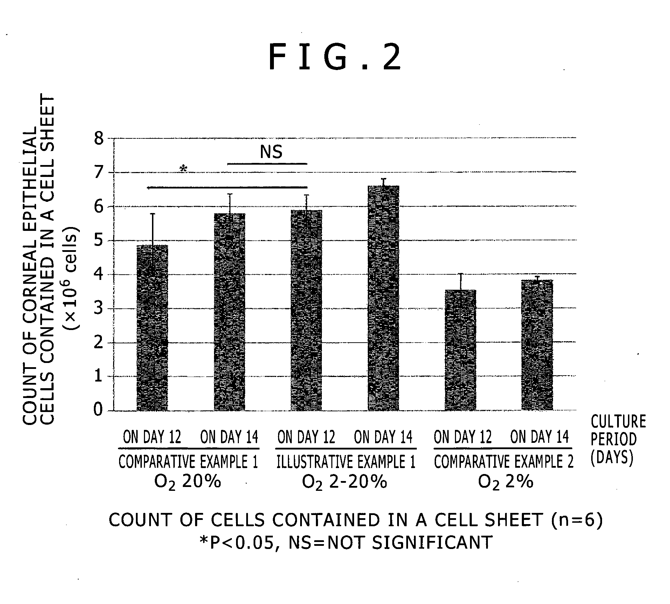

Fig. 2 is a view showing the counts of the illustrative example, cells contained in the cell sheets. In the first illustrative example, the cell count is at the same level on day 12 as that on day 14 in the comparative example 1, indicating that it is significantly greater than the cell count on day 12 statistically in the comparative example 1. In the comparative example 2, the cell count is significantly less on both day 12 and day 14 of culture than that on day 14 of culture in the comparative example 1. These findings suggest that the corneal epithelial cell sheets of the same quality level as that in the comparative example 1 may be produced faster than the first illustrative example by about two days. Moreover, the finding of the comparative example 2 suggests that the stratification of the cells do not progress when the cells are cultured at a low oxygen level in the stratification process.

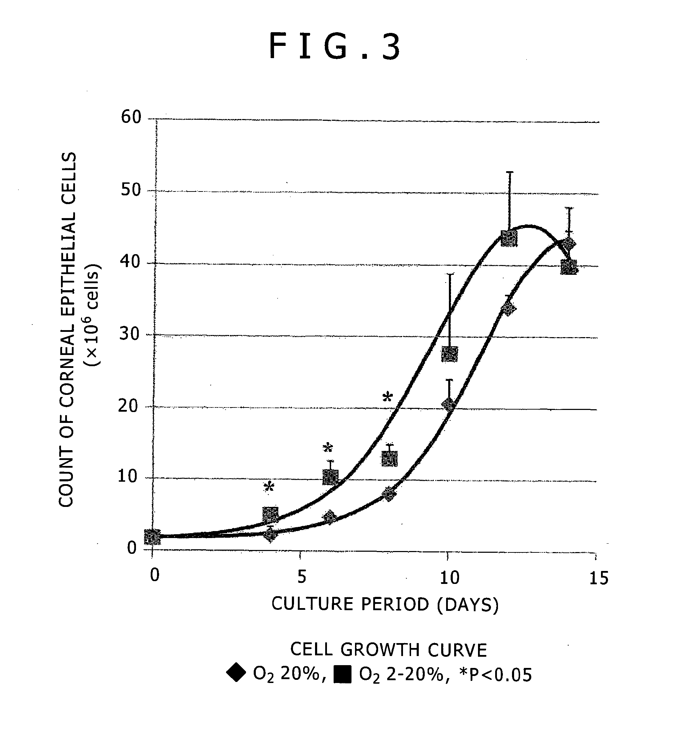

Fig. 3 is a view showing the results of quantitative analysis of cell counts in the first illustrative example and in the comparative example 1. In the first illustrative example, the cell count increased on day 4 or later of culture compared with that in the comparative example 1, in which the cells were cultured at 20% of O₂ level. This result supports the results shown in Fig 1 and Fig. 2.

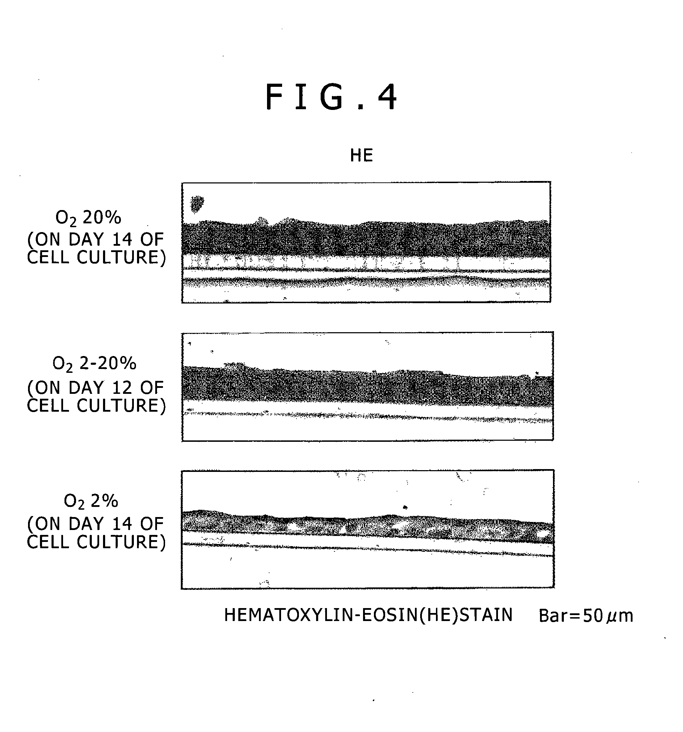

Fig. 4 is a view showing the tissue-stained images on day 12 of culture in the illustrative example 1, and on day 14 of culture in the first and comparative example 2s. In the first illustrative example, the tissue in the first illustrative example, of which quality was equal to that in the comparative example 1, was formed by two to six layers. In the comparative example 2, thin cell sheets were formed by one to two layers of cells. As known from the above results, the cells continuously cultured at low oxygen level are not smoothly stratified; however, when the oxygen level is changed to the normal level, the stratification process may be facilitated.

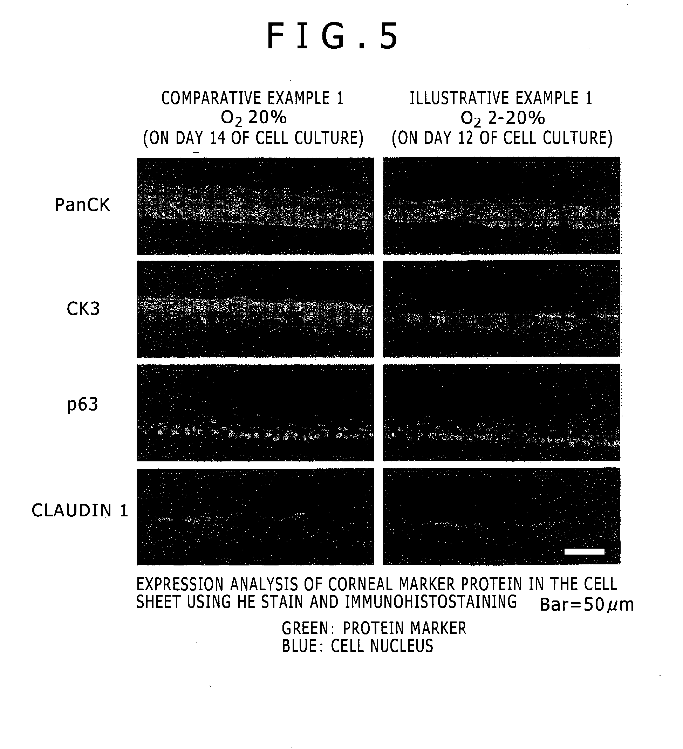

Fig. 5 is a view showing the immunostained images of the cells on day 12 in the first illustrative example and on day 14 in the comparative example 1 to assess the corneal epithelial cell sheets. The result of assessment demonstrated that CK protein family, an epithelial tissue-marker, was expressed in all the cells on day 12 in the first illustrative example. The CK3 protein, a differentiated corneal epithelial cell marker, was expressed in the details of the cells excluding their basal layer. Claudin 1, which is a protein related to a tight junction and important for the corneal barrier function was expressed in the uppermost surficial cells. The above results are the same as those on day 14 in the comparative example 1, suggesting that the obtained cell sheets serve as corneal epithelial tissues.

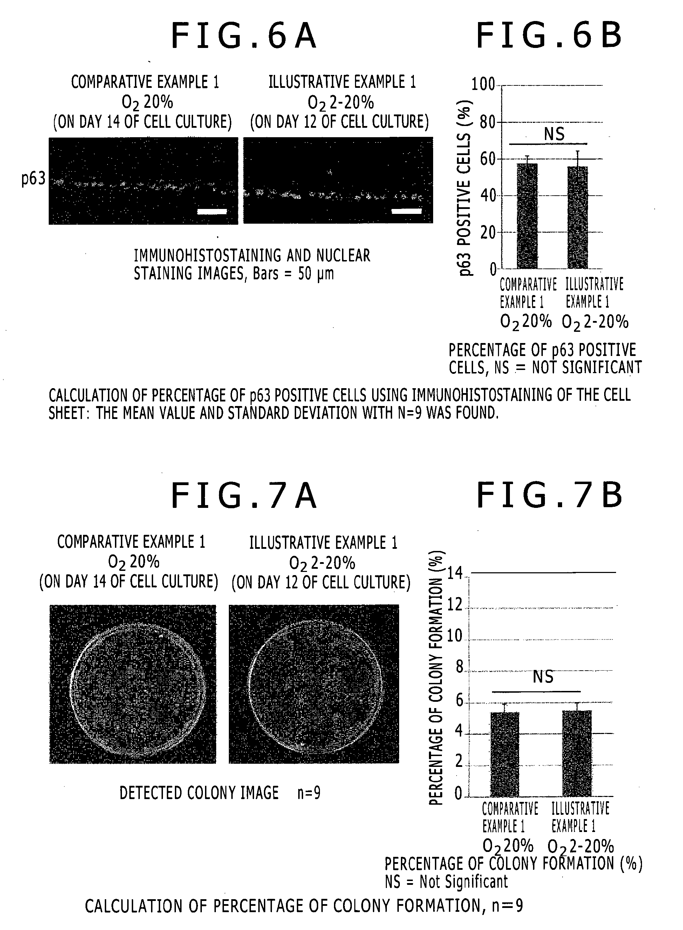

The existence of stem/progenitor cells in the cell sheet provides an important index for the cell growth progress after seeding. Fig. 6A is a view showing the p63 (stem/progenitor cell marker protein) tissue-stained images on day 12 in the first illustrative example and on day 14 in the comparative example 1, and Fig. 6B is a graph showing the rate of cells with p63-positive on the same days. In both the first illustrative example and the comparative example 1, p63 was expressed in the basal cells and the rate of p63-positice cells in the first illustrative example was at the same level as that in the comparative example 1. Figs. 7A and 7B are views showing the images of colony formation, which implicates the existence of stem/progenitor cells on day 12 in the first illustrative example and on day 14 in the comparative example 1, and the rates of colony formation in each of the examples. The rate of colony formation was at the same as that in the comparative example 1. The above results suggest that as in the comparative example 1, sufficient quantities of stem/progenitor cells exist in the cell sheets.

The cells are known for their different gene expression modes at the low oxygen environment. To examine a difference in gene expression level in the cell sheets between the first illustrative example, in which the sheets were produced through low oxygen culture, and the comparative example 1, exhaustive analysis of gene expression was carried out using a microarray. Comparison of luminescent intensity indicating the gene expression level between the first illustrative example and the comparative example 1 demonstrated that with correlation factor r=0.99, a strong correlation was observed and no significant variation was observed in a gene group known for its variable expression level during low-oxygen culture.

All the results shown in Figs. 1 to 8 indicate that the regenerated tissues may be produced in a short time period in the example 1 compared with that in the comparative example 1, which is a conventional method, and the quality of the produced tissues is at the same level as that in the comparative example 1. The culture condition of 2% of low O₂ level and 20% of normal O₂ level shown in this embodiment is one example illustrating the method for carrying our this embodiment and at O₂ level lower than 20%, the cells may be cultured in a shorter time period than in the comparative example 1. The result of verifying the above principle clarified that in particular, at O₂ level equal to or higher than 1% and lower than 15%, the cells would be cultured at a higher rate of growth in a shorter time period during the self-replication period. At O₂ level of 5%, especially, the cells could be cultured in the shortest time period.

It was shown that during the differentiation period, the differentiation of the cells is suppressed at a lower O₂ level but at O₂ level equal to or higher than 15%, the cells differentiated. Note that the O₂ level need to be at least lower than 60%, because at too high O₂ level, the differentiation tends to be over-suppressed, for example at 60% of O₂ level, no differentiation clearly occurs with high cell lethality. Moreover, it was clarified that at 02 level lower than 30%, the differentiation of the cells would be likely to occur. It is preferable that at 20% of O₂ level, namely the normal O₂ level, which is suitable for reducing reliably the cell culture time period, the cells are cultured. Hereinafter, the above experiments are more specifically explained.

### Method for culturing rabbit corneal epithelial cells

The 6-well culture inset type and 6-well plate type were used as cell culture containers. On the day before the corneal epithelial cells were cultured, NIH-3T3 cells treated with mitomycin C (10 µg/ml) at 37°C for 2 hours were seeded in the 6-well plate container as feeder cells at a seeding rate of 2 x 104/cm². On the following day of seeding of NIH-3T3 cells, corneal epithelial cells were collected from the corneal ring of the rabbit eyeball acquired from Funakoshi in the usual manner and seeded in the 6-well cell culture insert at a seeding rate of 2 x 104/cm². A KCM medium containing 5% FBS used for epithelial cells was used as a culture solution. The culture solution was exchanged in the upper and lower layers of the cell culture containers at the intervals of once day 5, 7, and 9-14 after culture was initiated. During the cell culture period, the cell growth progress was observed under the phase-contrast microscope.

### Method for measuring the count of the cells in the corneal epithelial cell sheets

The count of the cells contained in the cell sheets under the individual cell culture conditions and the count of the cells under growth were determined based on the amount of DNA. The method for calculating the count of the cells contained in the produced cell sheets is described below. First, Dispase (200 U/ml) was injected into the lower layer of the cell culture container and treated at 37°C for 60 minutes. After treatment, the Dispase was peeled away from the culture surface of the cell sheets with tweezers. The cell sheets with Dispase peeled away was treated with 0.25% trypsin at 37°C for 10 minutes and the cell count under growth was determined as described below; in this case, the count of the cells contained in the cell sheets as a suspension (TC10 boi-rad) was found. On days 4, 6, 8, 10, 12, and 14 of culture, the cells were collected and the count of the cells was calculated based on the luminescent intensity of the sample and the luminescent intensity of the sample, for which cell count was known, using a DNA determination kit (primary cell) (n=3).

### Methods for preparing the sections of the corneal epithelial tissue, staining the tissue section, calculating the rate of p63-positive cells, and calculating the rate of colony formation

The tissues were frozen-embedded on day 12 of culture in the first illustrative example, on day 17 in the first and comparative example 2s in the usual manner. 10 µm-thickness microtome sections were prepared from the frozen-embedded tissues. Using the prepared sections, HE-staining and immunostaining were carried out in the usual manner. For immunostaining, anti pan-CK antibody (clone name: Kspan1-8) anti CK3 antibody (AE5), anti clausin 1 antibody (A10), anti p63 antibody (4A4) were used. In the first illustrative example and the comparative example 1, the count of p63-positive cells / total cell count was calculated from five sections to obtain the rate of p63-positive cells. To find the rate of colony formation, 2,000 cells from the cell sheets, which have been prepared into a cell suspension using 0.25% trypsin, were seeded at the NIH-3T3 cell seeding rate of 2 x 104/cm². The cells were seeded on the 10 cm dishes and cultured in the KCM medium for about 10 days.

### Exhaustive analysis of gene expression

The total RNA was extracted from the cell using RNeasy mini kit (Qiagen). Using the Rabbit Oligo DNA Microarray (Agilent), the expression levels of about 30,000 genes were found in terms of luminescent signal intensity. (n = 3). The signal strength between the arrays was corrected using the 75 percentile shift method for normalization. Using the average value of n = 3, the gene expression levels were compared between the first illustrative example 1 and the comparative example 1 to find a correlation factor r.

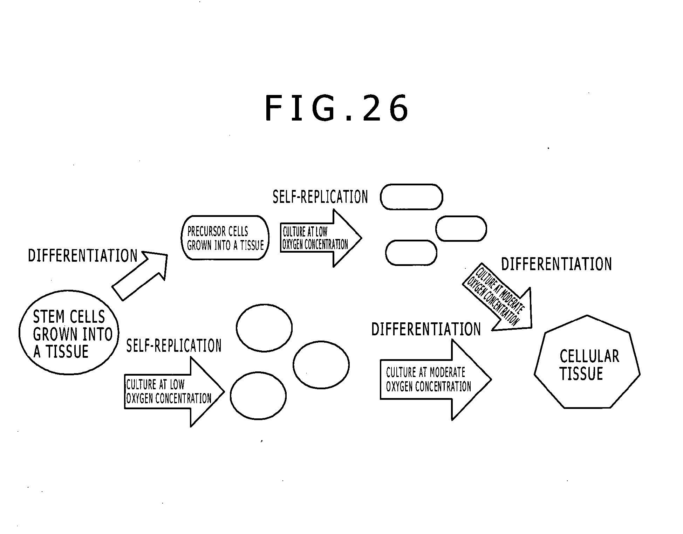

By reference to Fig. 26, the range of cell species, to which the above method may be applicable, is explained. The cell species, to which the above method is applicable, include the stem cells capable of forming tissues and the progenitor cells produced through the differentiation of the stem cells. After self-replication and growth to the given degree, these cells initiate differentiation at the normal oxygen level. Generally, the differentiation of the cells tends to be suppressed at low oxygen level. In other words, change from low oxygen level to normal oxygen level triggers the initiation of differentiation of stem cells capable of forming tissues and the progenitor cells.

Second, the method for changing the oxygen level during the process of culturing the stem cells capable of forming tissues and the progenitor cells is specifically explained. First, the cells seeded in the culture space such as a culture container are cultured at low oxygen level. During the self-replication period, the cells repeat growth through self-replication more rapidly than at the normal oxygen level. The whole size of the stem cell or progenitor cell associated into a larger one through growth, the size of a single cell, or the growth rate may vary depending on the type of the stem cells or the progenitor cells to be cultured. For this reason, it is preferable that the sizes of the cells obtained from self-replication are appropriately set depending on the cell types to change from low oxygen level to the normal oxygen level. The cells, when exposed to the normal oxygen level, initiate differentiation and form a tissue.

The cell growth progress may be determined based on the coverage of cells on the culture surface. The cells initiate growth through self-replication and spread over the culture surface in the culture space, in which they were seeded. When the coverage of cells on the culture surface reaches 100%, as explained with respect to the above principle, the cells are in the confluent state; at this point, the low oxygen level is changed to the normal oxygen level (20%). Note that the coverage of cells, by which the timing of changing the oxygen level is determined, may be set to any of rates such as 80% and 90% depending on the characteristics of the cell species.

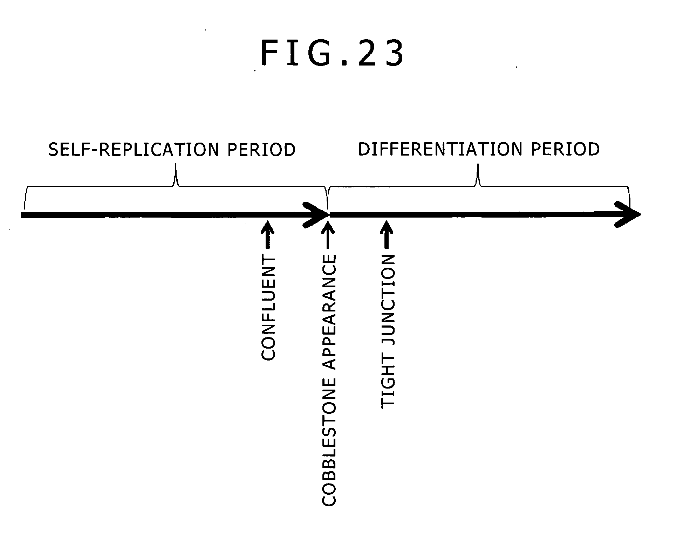

The oxygen level may be effectively changed at the point when the cells are in the confluent state as explained above, as well as the cobblestone state. As shown in Fig. 23, the cobblestone state of cells exists during the period from the confluent state to the initiation of differentiation; changing the oxygen level in this cobblestone state of cells enables the cells to be cultured at low oxygen level up to the immediately before the initiation of differentiation, achieving cell culture in a short time period compared with that in the confluent state.

Moreover, after changing the oxygen level, as shown in Fig. 23, by checking whether the tight junction phenomenon has occurred during the differentiation period following the cobblestone state, the quality of the cells cultured after changing the oxygen level may be assessed. The tight junction is a structure with intercellular gaps closed with claudin and occluding, which are membrane-spanning proteins. Through tight conjunction state, the cultured cells control paracellular pathways of dissolved substances, ions, and water. Specifically, it may be verified, by checking that the cells are in the tight junction state, that the cells are cultured with no foreign substances such as dissolved substances and contaminants.

The method for controlling the oxygen level may be controlled by means of oxygen supply fed into the cells to be cultured. For example, to increase the oxygen level of a gas supplied to the cells, a larger amount of oxygen may be supplied or a less amount of nitrogen or carbon oxide may be supplied. In contrast, to decrease the oxygen level, a less amount of oxygen may be supplied or a larger amount of nitrogen or carbon oxide may be supplied.

### Example 2

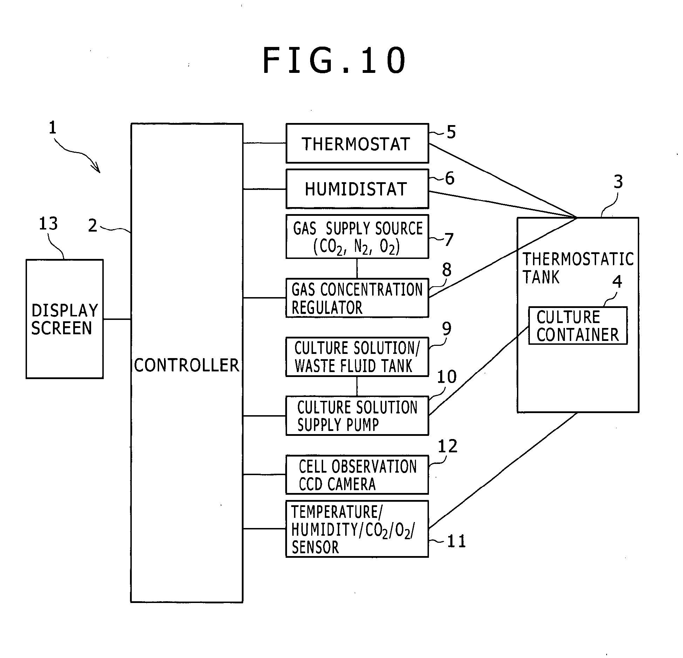

With respect to the example 2, an automatic cell culture device equipped with a function for controlling the oxygen level automatically, based on the principle explained with respect to the example 1, is explained be reference to Figs. 10 to 13. Note that taking an example of a controller 2 having an internal step of carrying out calculation relating to the oxygen level, the example 2 and subsequent embodiments are explained but software and a CPU are not limited to them. The software may be installed in an external computer or a gas temperature adjusting part.

To control the oxygen level, the user may observe visually the cells in the cell culture device to change the oxygen level appropriately; alternatively, any of parts for taking images of the observed cells, such as a CCD camera 12, may be disposed to view the taken images on the display screen 13 so that the user changes the oxygen level based on the displayed images. However, the display screen 13 is not limited to a visual device and any of audible devices for sounding a warning, such as a buzzer, may be used.

To control the oxygen level automatically, it is preferable that the cell state is automatically recognized to determine the timing suitable for changing the oxygen level. First, the images of the cells are taken during the culture process using a CCD camera 12; a controller 2 obtains the images of the cells and detects the cells from the obtained image data, and digitizes the image data based on the brightness levels of the images for monochrome or grayscale presentation to calculate the coverage of cells in the images. After data is obtained at several points on the culture surface, if the coverage of cells is 100%, the oxygen level is changed from low oxygen level to the normal oxygen level. If the coverage of cells has not reached to the given level, the oxygen level is not changed, the culture process is continued at low oxygen level, and the above steps are repeated. Then, the coverage of cells reaches 100%, the oxygen level is changed.

Since the coverage of cells increases as cell replication advances, the oxygen level is preferably changed in the confluent state with the coverage of cells close to 100% around the final step of the self-replication process. Note that the oxygen level may be set to any of levels such as 80% or 90% depending on the cell culture progress, the surface of the culture area, in which the cells are actually cultures, etc. Alternatively, if the cells do not reach the confluent state depending on the cell types, the timing for changing the oxygen level may be set based on the size of the cells until self-replication is complete, or the coverage of cells.

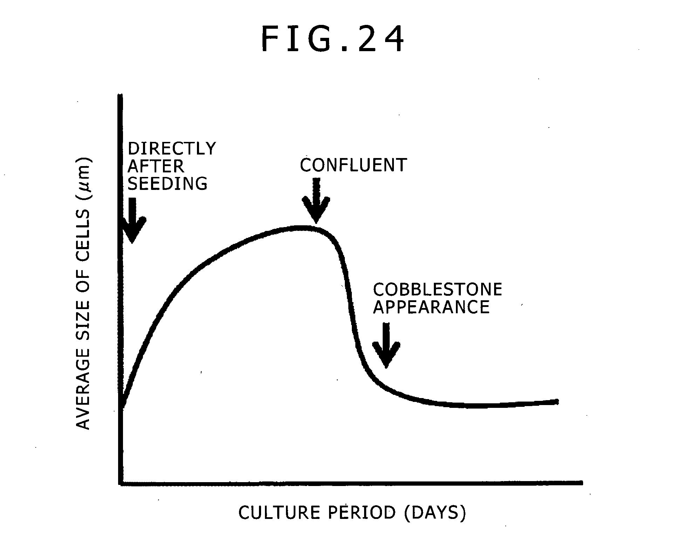

The timing may be determined based on the average size of the cells in the confluent state. As shown in Fig. 24, since the cells attached on the culture surface grow while extending their pseudopod, the cell size becomes larger than that immediately after seeding, the average size of cells taking the maximum value around the confluent state. Then, the cell size reduces because the cell count per area increases as the cells progress toward the cobblestone state; accordingly, the average size of the cells gradually decreases and the shapes of the cells are fixed in the cobblestone state and later, resulting in certain size of cells.

The CCD camera 12 takes a plurality of time-series images and the controller 2 calculates statistic data on the cell size based on the plurality of images using a plot file, etc. Moreover, based on the calculated statistic data, the average size of the cells for each image is calculated to compare the average size of the cells among the time-series data. The time-series changes in average cell size are determined to identify the timing when the cell size reaches the largest level.

Moreover, the controller 2 changes the oxygen level after the timing takes the identified largest value, namely after the timing when the cells proceed to the confluent state. In this case, information such as the maximum size of the cells and the identified time period, during which the oxygen level is changed, may be viewed on the display screen 13 to prompt the user to change the oxygen level.

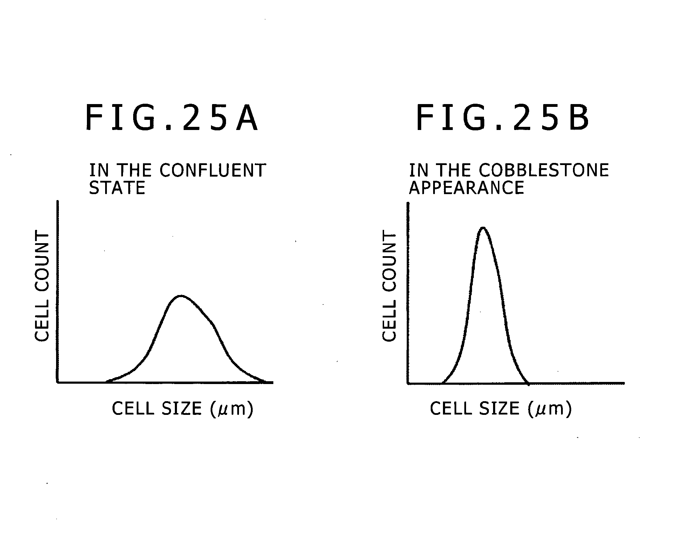

The method for identifying the largest value of cells is not limited to the aforementioned method. Alternatively, a certain value is set for each cell species and the oxygen level is changed at the timing when the cells carry out self-replication up to the level exceeding this value. Alternatively, instead of the average size of cells, as shown in Fig. 25A, the time-series change in size distribution is analyzed on the images to set the largest peak in the distribution as the maximum value, or a variance for changing the oxygen level may be set in advance.

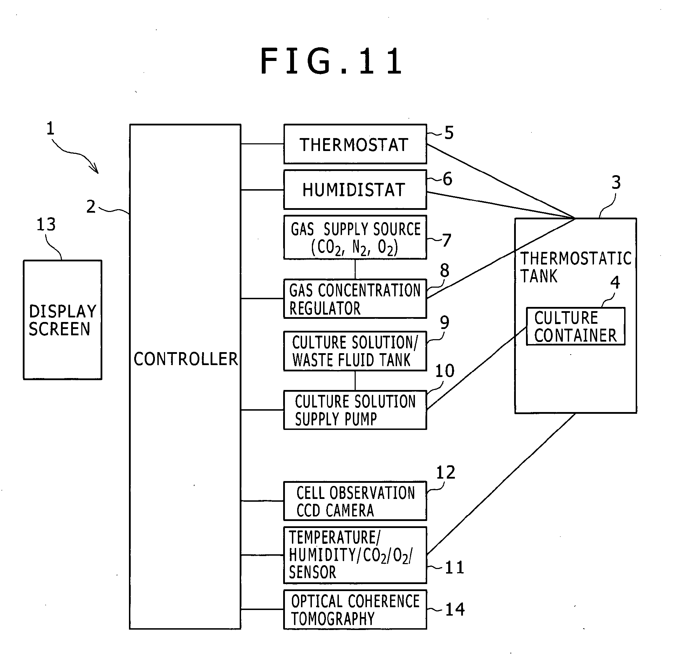

In addition to the method for determining the timing of changing the oxygen level on the cell images as described above, the timing of changing the oxygen level may be determined by analyzing the cell state using an optical coherence tomography as shown in Fig. 11. With the method for determining the timing on the cell images, it is not known whether the areas, which are not observed, have been in the confluent state in some cases because only several points are observed on the culture surface. On the other hand, with the method for analyzing the cell state using the optical coherence tomography 14, the areas, in which no cells are observed, may be detected on the whole culture surface. The optical coherence tomography 14 irradiates one of two split infrared lights onto the sample to induce coherence between the irradiated light and the other split light, achieving the images of the surface and cross-section of the tissue over the whole medium surface.

A light source disposed at the optical coherence tomography 14 may be equipped with a driving part capable of varying the irradiation position of the infrared light irradiated from the light source. Using this driving part, the cross-sectional image of the culture surface is obtained in one direction. Moreover, by obtaining a plurality of cross-sectional images while moving the driving part vertically relative to the abovementioned one direction, the cross-sectional images over the whole culture surface may be obtained. The vertical interval between the cross-sectional images to be obtained is preferably narrower than the cell size to avoid omission of areas, in which no cell is observed. Alternatively, since the cell size is analyzed by an image analysis method described later and the like, the interval between the cross-sectional images to be obtained may be determined depending on the cell growth progress. The obtained images may be viewed on the display part 16. Only the images containing the areas with no cell may be displayed, or instead of displaying, a buzzer may be caused to sound to inform of cells being not found. In this way, based on cross-sectional information on the cells obtained from the optical coherence tomography 14, it may be determined whether the cells are found or not. The controller 2 changes the low oxygen level to the normal oxygen level, if the cells are in the confluent state, based on the result of detection by the optical coherence tomography 14.

The aforementioned method using cell images and the method using an optical coherence tomography may be used together, making automatic control of the oxygen level more reliably. For example, when the coverage of cells on the culture surface is equal to or higher than the given value (e.g., 100%), and the same thickness as that of the cell is observed on the whole culture surface (e.g., 100%), the oxygen level may be changed to determine more reliably whether the cells are in the confluent state.

As explained above, it is possible to determine whether the cells are in the confluent state and change the oxygen level. It is preferable that the oxygen level is changed when a given time elapses after the cells enter the influent state to reduce the culture period. However, if the given time has passed, the cells enter the cobblestone state explained with respect to the example 3 in detail. To avoid this problem, the timing of changing is set in advance considering the possible occurrence of the cobblestone state, making it possible to culture the cells in a shorter time period.

With respect to the example 2, the scenario, in which the calculating step is incorporated in the controller 2, has been explained: Alternatively, the calculation step may be incorporated in a gas concentration adjusting part 8 independent of the controller 2 to control the change of the oxygen level.

To control the oxygen level, the controller 2 is used to control individual gas supplies from a gas supply source at the gas concentration adjusting part 8. For example, to increase the level of oxygen supplied to the cells, oxygen may be supplied in greater amount, or nitrogen or carbon oxide may be supplied in less amount. In contrast, to decrease the oxygen level, oxygen is supplied in less amount. Alternatively, nitrogen or carbon oxide may be supplied in greater amount.

### Example 3

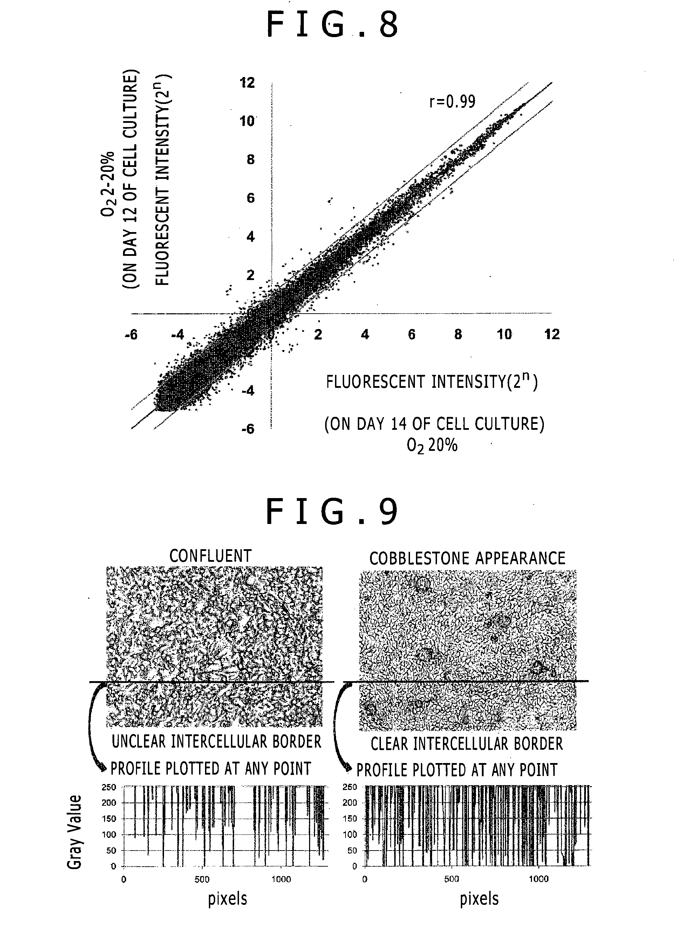

The oxygen level may be changed when the cells are in the cobblestone state, in which the density of the cells is increased and the volume of each cell is reduced, resulting in tightly arranged cells, rather than the confluent state. Stratification, part of differentiation, occurs after the cells leave from the cobblestone state; accordingly, the oxygen level is changed, when the cells are in the cobblestone state, to produce the desired tissues in a shorter time period. The controller 2 first processes the images of the cells so that the individual cells may be clearly identified, after the coverage of cells reaches 100%, based on the result of cell analysis explained with respect to the example 2. Then, the controller 2, as shown in Fig. 9, calculates a change of brightness on one line arbitrarily set on the image in terms of signals. Based on the distribution of the signals, it is determined whether the signals are regularly detected at the intervals of about 5 to 15 µm equivalent to the sizes of the cells in the cobblestone state. If so, the cells are in the cobblestone state. At this point, the low oxygen level is changed to the normal oxygen level. If not so, namely if it is determined that the sizes of the cells are not equivalent to those in the cobblestone state based on the detected signals indicating the intracellular distance, the culture is continued at the low oxygen level and the above procedure is repeated.

Alternatively, it may be determined whether the cells are in the cobblestone state based on the average size of the cells or the distribution of the cells from the distribution of signals on the images taken by a CCD camera 12.

The brightness is relatively decreased around the peripheries of the cells on the images, while the bright ness of the individual cells is relatively increased. Specifically, the brightness of most of the cells is high because at the early stage of culture, the cell count is less, resulting in high average brightness of the images. On the other hand, as the cell count increases, the peripheries (low-brightness pasts) increases, leading to darker average brightness of the images.

After the cells are in the confluent state on the culture surface, the cells are more tightly arranged on the culture surface through growth as they proceed toward the cobblestone state. At this point, the cell count increases followed by an increase in area of the peripheries of the cells, the average brightness continuing to lower.

The final cobblestone state of cells indicates the saturated self-replication of the cells on the culture surface and during the period from the occurrence of the cobblestone state to the initiation of differentiation, the average brightness of the images remains unchanged. For this reason, the controller 2 may determine the time when the average brightness of the images reaches a constant level to be the cobblestone state and change the oxygen level.

Alternatively, the use of the average size of the cells, as explained with respect to the example 2, makes it possible to determine whether the cells are in the cobblestone state. As shown in Fig. 24, in the cobblestone state, in which the cells are compacted, a time-series change in cells remains unchanged. During this period, in which the time-series change remains unchanged, the oxygen level is changed. The procedure for calculating this period at the controller 2 is the same as that explained with respect to the example 2.

Alternatively, it may be determined whether the cells are in the cobblestone state at the controller 2 based on analysis of the distribution of the cell sizes. As shown in Fig. 25, the sizes of the cells during the self-replication period are larger than those in the cobblestone state. Moreover, variation may occur in progress of culture in individual culture areas on the culture surface; thereby, the areas, in which variance occurs, increase. The sizes of the cells and the distribution of the cell sizes decrease gradually as the cells proceed toward the cobblestone state, and once they have entered the cobblestone state, the sizes of the cells reach their minimal level and remains unchanged during the self-replication period.

As mentioned above, the controller 2 may determine whether the cells are in the cobblestone state to change the oxygen level, when the state of the cells moves to the area, in which the distribution of the cell sizes is the smallest, or when the range of variance in the distribution is the smallest, or when the time-series change in distribution of cell sizes, or when any of these conditions are combined.

The oxygen level may be adjusted by causing the controller 2 to control the gas concentration adjusting part 8 to change the oxygen supply as in the example 2. The detailed explanation is omitted to avoid the duplication. The images taken by a CCD camera 12 and data calculated at the controller 2 as described above may be viewed in the display screen 13. Moreover, the timing, when the oxygen level is changed, may be viewed on the display screen 13 to prompt the user to change the oxygen level.

### Example 4

With respect to the example 4, the method for changing the oxygen level across the whole culture tank, a culture space, is explained by reference to the method for cell culture explained with respect to the example 1 and the method for automatically controlling the oxygen level explained with respect to the examples 2 and 3. Note that the explanation of function of the display screen 13 is omitted to avoid duplication.

Fig. 10 is a schematic diagram showing the configuration of the cell culture device 1. As known from this figure, the individual parts controlled by the controller 2 are connected to a thermostatic tank 3 and a culture container 4 in the thermostatic tank 3. The controller 2 is connected to: a the temperature adjusting part 5 for controlling the temperature of the thermostatic tank 3; a humidity adjusting part 6 for adjusting the humidity in the culture container; a gas concentration adjusting part 8 with a gas supply source 7 for controlling the gas level in the culture container; a culture solution supply pump 10 with a supply tube , which is connected to a tank 9 for storing the culture solution and waste liquid, for automatically exchanging the culture solution in the culture container; a temperature / humidity / CO₂ / O₂ sensor 11 for controlling the actions by the individual parts; a CCD camera 12 for cell observation; and a display screen 13. Further, the temperature adjusting part 4, the humidity adjusting part 5, and the gas concentration adjusting part 7 are connected to the thermostatic tank 2 and the culture solution supply pump 8 is connected to the cell culture container 3. In the above configuration, oxygen is supplied in the thermostatic tank; accordingly, in the closed culture container 3, it is preferable to install a gas-permeable porous membrane made of any of materials such as polystyrene, polycarbonate, polyethylene terephthalate, and polymethyl pentene, preferably polycarbonate, polyethylene terephthalate, and polyimide. The diameter of the porous membrane is preferably less than 20 nm to avoid invasion of viruses and bacteria in the culture container. This size is based on the diameter 20 nm of parvovirus, the smallest one of known viruses.

Fig. 11 shows the configuration according to the example 4, in which the optical coherence tomography 14 according to the example 3. The optical coherence tomography capable of measuring the cross-sectional thickness may be applicable to non-invasive determination of the quality of the produced cell sheets whether they have differentiated.

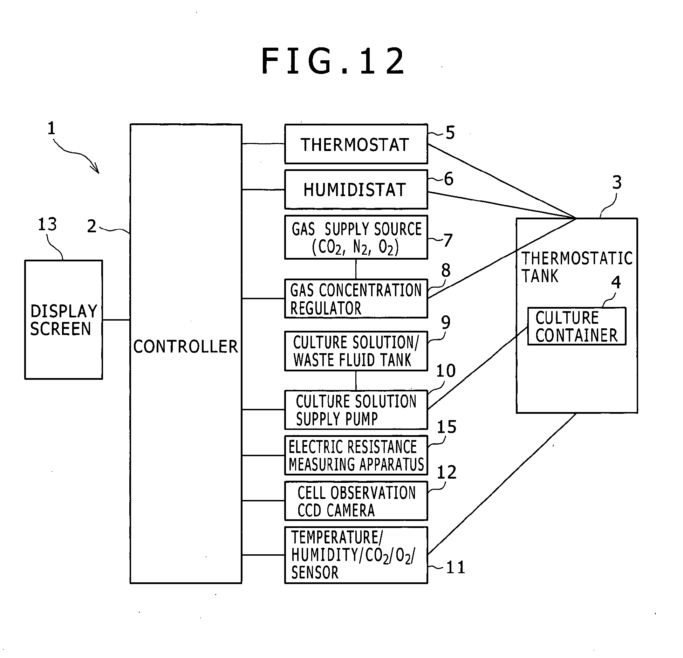

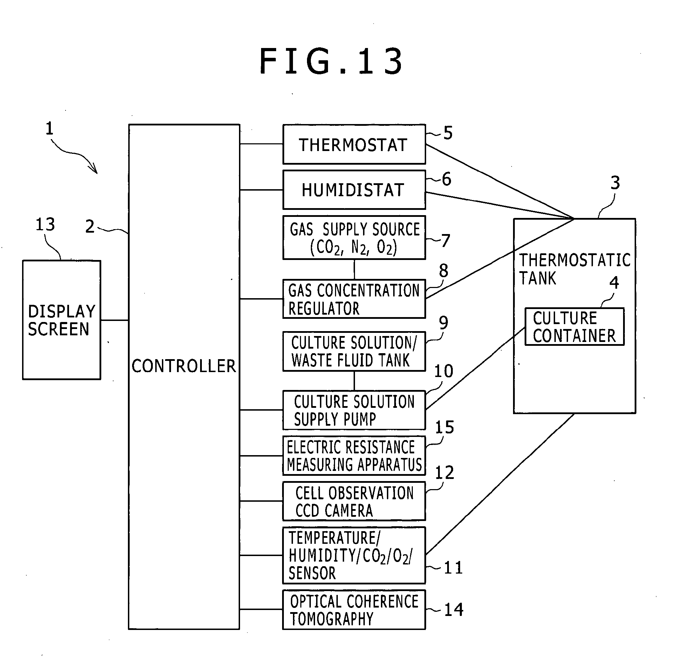

The non-invasive method for assessing the quality of the cell sheets includes the method for estimation using the electric resistance values, in addition to the method using use of the optical coherence tomography. The method for assessing the quality of the cell sheets based on the electric resistance values is explained below. The epithelial cells form a tight junction through tight association with each other. Once the tight junction among the cells has been formed, ion exchange between the cells is interrupted, causing resistance when a voltage is applied between the cells. Specifically, the tightly associating cells enter the cobblestone state; accordingly, it may be determined whether the tight junction has been formed based on the electric resistance values. Fig. 11 shows a variation of the configuration with the optical coherence tomography 14 attached according to the example 2 and Fig. 12 shows a variation of the configuration with the electric resistance measuring apparatus 15 attached according to the example 2. The controller 2 calculates the time-series change in resistance value measured at the electric resistance measuring apparatus 15 and based on the result of calculation, makes analysis to determine whether the electric resistance value exponentially changes. If the resistance value exponentially changes, the tight junction is determined to occur. Alternatively, the electric resistance measuring apparatus may be combined with the optical coherence tomography 14 as shown in Fig. 13 to verify the quality of the cultured cells.

To control the oxygen level, the oxygen supply to the thermostatic tank may be used. For example, to increase the oxygen level of the gas supplied to the cells, the controller 2 controls the gas concentration adjusting part 8 in order to supply oxygen in greater amount or nitrogen or carbon oxide in less amount. In contrast, to decrease the oxygen level, oxygen is supplied in less amount in to the culture container. Alternatively, nitrogen or carbon oxide may be supplied in greater amount.

### Example 5

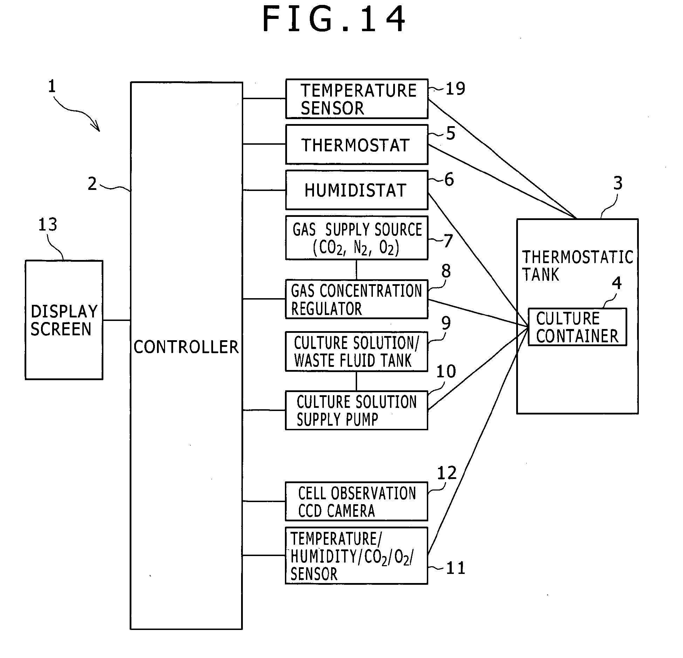

According to the example 4, the oxygen level in the culture tank is controlled. As shown in Fig. 14, according to the example 5, the temperature adjusting part, the humidity adjusting part, the gas adjusting part, and the temperature / humidity / CO₂ / O₂ sensor are connected the culture container to control the oxygen level therein. This scenario is explained with regard to the example 5.

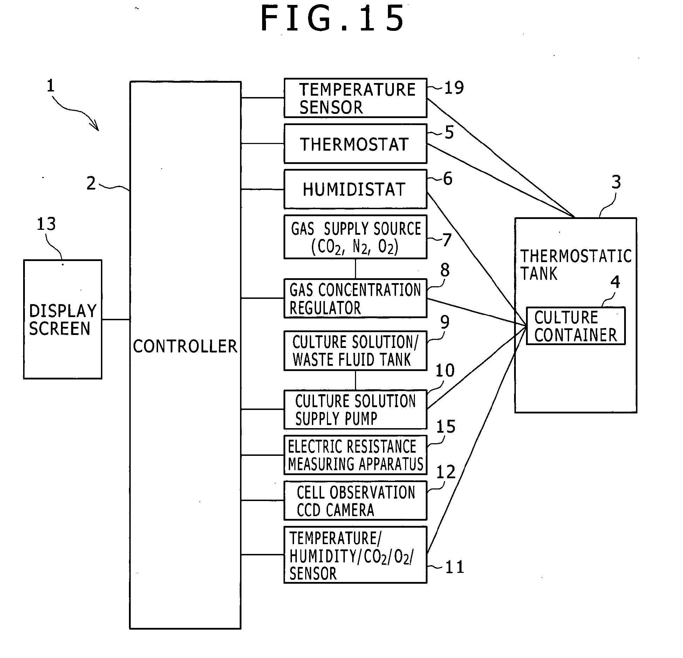

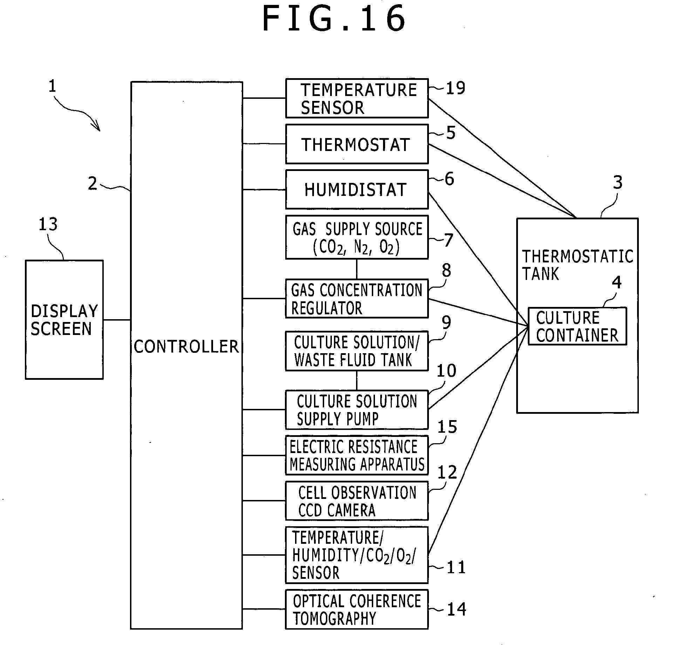

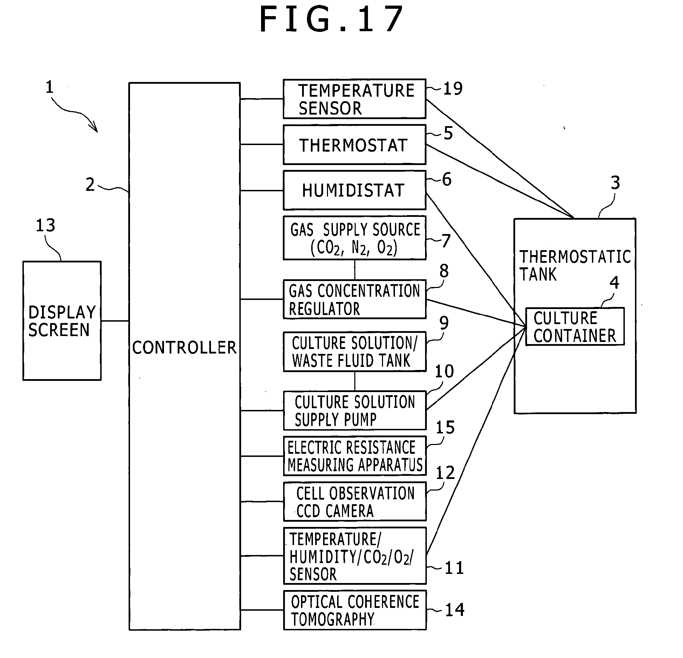

This configuration enables water vapor to enter the culture container via a gas supply port of the culture container. However, the culture tank, which has not completely sterilized in its entire space, has a risk of growth of fungus and bacteria if it is under the high temperature and high humidity environment. In contrast, the culture container, which has completely sterilized, has a less risk of growth of bacteria and fungus even in high temperature and high humidity environment. According to the example 5, the need for installing a special membrane to the culture container is eliminated, achieving the simplified process of manufacturing the culture container. Figs. 15 to 17 show the variation of the configuration according to the fifth embedment as in the example 4.

### Sixth Embodiment

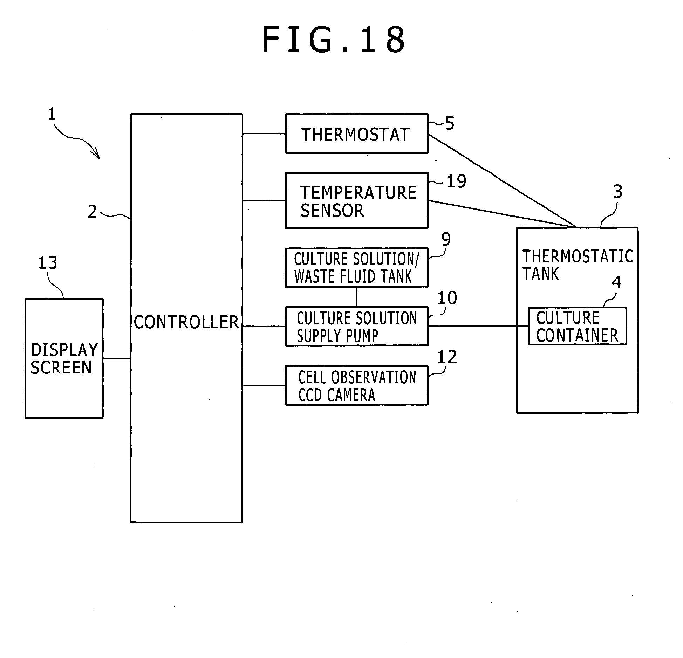

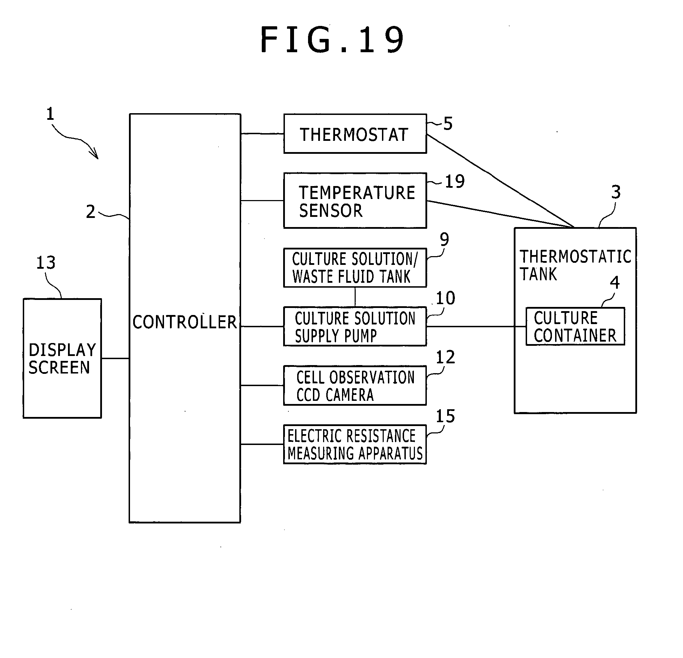

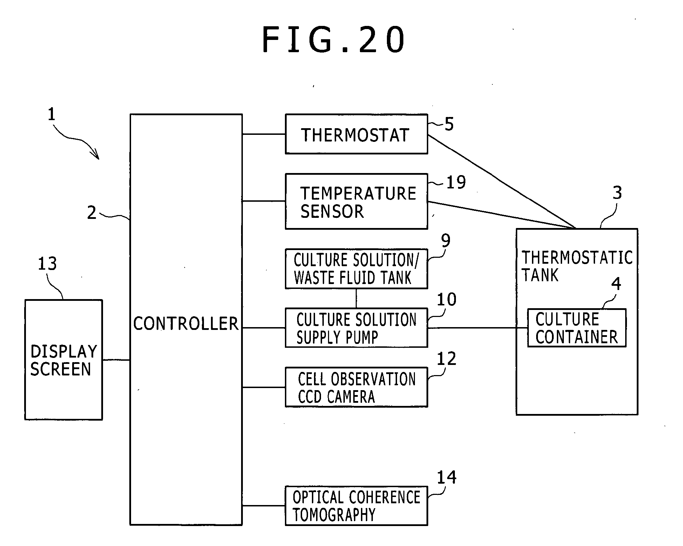

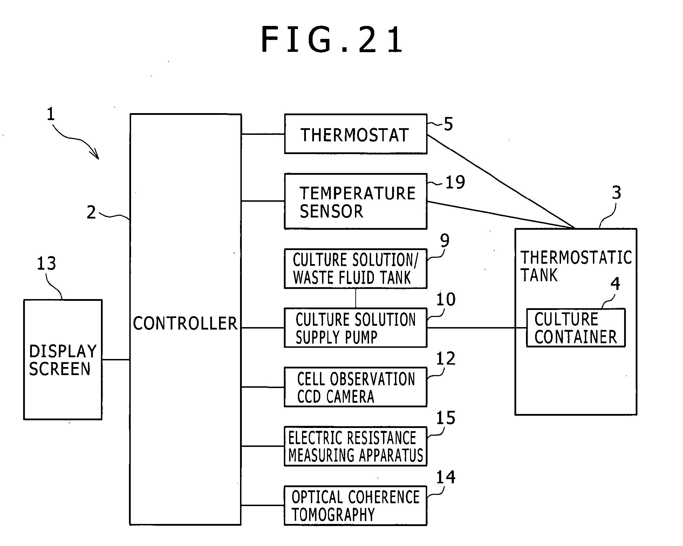

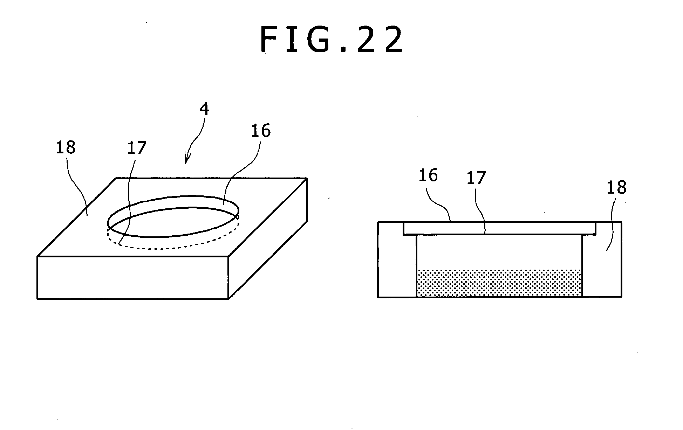

According to the examples 4 and 5, the oxygen level is controlled in the culture tank and the culture container and the permeability of the gas permeable membrane installed in the culture container may be varied. This configuration eliminates the need for supplying a low oxygen gas and water vapor into the culture container and has ability to control the oxygen level in the culture container. For example, as shown in Fig. 18, the configuration may be simplified because of the eliminated need for the humidity adjusting part, the gas adjusting part, and the CO₂ / O₂ sensor. Figs. 19 to 21 show a variation of the configuration. With respect to this configuration, the shape of the culture container is important. One example of the above configuration is shown in Fig. 22. Two glass-permeable membranes are installed to the frame body 18 of the culture container 4 and the outermost membrane suppresses the invasion of oxygen. This constraint membrane 16 structured into removable one reduces the oxygen level in the culture container. Some of the constraint membranes 16 are made of materials such as polyethylene terephthalate, PVA, nylon, nylon family, and silica-evaporated film family, which have been commonly used for medical drug and food packaging materials. The other type of membrane include the film with a special layer, called super barrier film, (Fuji Film), which are used for organic EL, electric paper, solar cell. The porous membrane 17 with a pore less than 20 nm in diameter explained with respect to the example 2 is used as an inner membrane. During the self-replication period, the cells are cultures with the constraint membrane 16 retained. This achieves the low oxygen culture environment. After the self-replication period, a mechanism has been incorporated for automatically removing the constraint membrane 16 to return the oxygen level back to its normal level; the controller 2 manipulates this mechanism; and the constraint membrane 16 is removed during the confluent or cobblestone state to change the oxygen level in the culture container.

To remove the constraint membrane 16, a manipulator with a driving part, etc. has been incorporate in the cell culture device and the controller 2 drives the manipulator to remove the constraint membrane 16. After the removal of the constraint membrane, the process proceeds to cell differentiation. However, the culture solution may vaporize because the inside of the culture tank is not under the high-humidity condition. To avoid this problem, a mechanism for automatically injecting the culture solution in the same amount as that vaporized has been incorporated in the cell culture container effectively to keep the volume of the culture solution constant.

The present invention may be summarized by reference to the aforementioned embodiments.

The cell culture device of the present invention has a culture area for culturing the stem cells or the progenitor cells used to produce tissues. The cell culture device has further an oxygen adjusting part for adjusting an oxygen supply in the culture area; a controller for controlling the oxygen adjusting part; and another controller for controlling a first oxygen supply in a first period, in the stem cells or the progenitor cells self-replicate, and a second oxygen supply in greater amount than that of the first oxygen supply in a second period, in which the stem cells or the progenitor cells differentiate, and further controlling the oxygen adjusting part to change the first oxygen supply to the second oxygen supply based on the growth progress through self-replication.

The cell culture device of the present invention has a container support for supporting a culture container having a culture area for culturing stem cells or progenitor cells used to produce tissues; an oxygen adjusting part for adjusting an oxygen supply in the culture container; a controller for controlling the oxygen supply; and another controller for controlling a first oxygen supply in a period, during which stem cells or the progenitor cells grow up until they come into contact with a culture surface of the culture area, and a second oxygen supply greater in amount than the first oxygen supply in a second period, during which the grown stem cells or the progenitor cells stratify on the culture surface, and further the oxygen adjusting part to change the first oxygen supply to the second oxygen supply based on a grow progress in the period, during which the stem cells or the progenitor cells grow up until they come into contact with the culture surface.

In the cell culture device, the first oxygen supply is equal to or higher than 1% and less than 15%.

In the cell culture device, the second oxygen supply is equal to or higher than 15% and less than 60%.

The cell culture device has further an imaging part for taking the images of the stem cells or the progenitor cells and the controller calculates the growth progress based on the images obtained from the imaging part.

The controller controls the oxygen adjusting part to change the first oxygen supply to the second oxygen supply when the stem cells or the progenitor cells grow up to a given area in the culture area through self-replication.

The cell culture device according to claim 5, wherein the controller controls the oxygen adjusting part to change the first oxygen supply to the second oxygen supply when a given time has passed after the stem cells or the progenitor cells grew up to the given area in the culture area.

The cell culture device has further a first surface for culturing the stem cells or the progenitor cells in the culture area and the growth progress is the coverage of the stem cells or the progenitor cells on the first surface.

The cell culture device according to claim 8, wherein it has further an imaging part for taking images of the stem cells or the progenitor cells and the controller calculates the coverage based on the images obtained from the imaging part.

The cell culture device has further a display screen for viewing the coverage.

The controller controls the oxygen adjusting part to change the first oxygen supply to the second oxygen supply based on the coverage.

The cell culture device according to claim 1, wherein it has further an imaging part for taking images of the stem cells or the progenitor cells and the controller calculates a time-series change in average size of the stem cells or the progenitor cells based on the images obtained from the imaging part.

The cell culture device has further a display screen for viewing the time-series change.

The controller, based on the time-series change, identifies the period after the average size of the stem cells or the progenitor cells reaches its maximum level, out of the whole culture period, during which the stem cells or the progenitor cells self-replicate, and controls the oxygen adjusting part to change the first oxygen supply to the second oxygen supply.

The controller controls the oxygen adjusting part to change the first oxygen supply to the second supply in the period, during which the average size of the stem cells or the progenitor cells is constant after the average size reaches its maximum level.

The cell culture device has further an optical coherence tomography for irradiating a first light capable of permeating the stem cells and the progenitor cells and a second light capable of reflecting on the surfaces of the stem cells or the progenitor cells. The optical coherence tomography calculates the coverage based on the interference between the first and second lights.

The cell culture device has further an electric resistance measuring part for measuring the electric resistance values for the stem cells or the progenitor cells.

A method for cell culture of the present invention involves the steps of : culturing the stem cells or the progenitor cells at a first oxygen level in a first period of the whole culture period, in which the stem cells or the progenitor cell self-replicate; and changing the first oxygen supply to the second oxygen supply in greater amount than that of the first oxygen supply based on the growth progress to culture the stem cells or the progenitor cells differentiate at the second oxygen supply in the second period of the whole culture period.

### Industrial Applicability

The present invention is useful as the method for cell culture and as the cell culture device.

### List of Reference Signs

- 1: Cell culture device
- 2: Controller
- 3: Thermostatic tank
- 4: Culture container
- 5: Temperature adjusting part
- 6: Humidity adjusting part
- 7: Gas supply source
- 8: Gas concentration adjusting part
- 9: Culture solution, waste fluid tank
- 10: Culture solution supply pump
- 11: Temperature / humidity / gas sensor
- 12: Cell observation CCD camera
- 13: Display screen
- 14: Optical coherence tomography
- 15: Electric resistance measuring apparatus
- 16: Percolation membrane
- 17: Porous membrane
- 18: Frame body
- 19: Temperature sensor

## Claims

1. A cell culture device having a culture area for culturing stem cells or progenitor cells used for producing tissues, the device comprising:
an oxygen adjusting part that adjusts an oxygen supply in a culture area;
a controller that controls the oxygen adjusting part; and
another controller that controls a first oxygen supply during a first period, in which the stem cells or the progenitor cells self-replicate, and a second oxygen supply greater in amount than the first oxygen supply during a second period, in which the stem cells or the progenitor cells differentiate, out of the whole culture period, and that controls the oxygen adjusting part to change the first oxygen supply to the second oxygen supply based on the growth progress of the stem cells or the progenitor cells through self-replication.

2. A cell culture device comprising:
a container support that supports a culture container having a culture area for culturing stem cells or progenitor cells used to produce tissues;
an oxygen adjusting part that adjusts an oxygen supply in the culture container;
a controller that controls the oxygen supply; and
another controller that controls a first oxygen supply in a period, during which stem cells or the progenitor cells grow up until they come into contact with a culture surface of the culture area, and a second oxygen supply greater in amount than the first oxygen supply in a second period, during which the grown stem cells or the progenitor cells stratify on the culture surface, and that controls the oxygen adjusting part to change the first oxygen supply to the second oxygen supply based on a grow progress in the period, during which the stem cells or the progenitor cells grow up until they come into contact with the culture surface.

3. The cell culture device according to claim 1, wherein the first oxygen supply is equal to or higher than 1% and less than 15%.

4. The cell culture device according to claim 1, wherein the second oxygen supply is equal to or higher than 15% and less than 60%.

5. The cell culture device according to claim 1, further comprising:
an imaging part that takes images of the stem cells or the progenitor cells,
wherein the controller calculates the growth progress based on the images obtained from the imaging part.

6. The cell culture device according to claim 5, wherein the controller controls the oxygen adjusting part to change the first oxygen supply to the second oxygen supply when the stem cells or the progenitor cells grow up to a given area in the culture area through self-replication.

7. The cell culture device according to claim 5, wherein the controller controls the oxygen adjusting part to change the first oxygen supply to the second oxygen supply when a given time has passed after the stem cells or the progenitor cells grew up to the given area in the culture area.

8. The cell culture device according to claim 1, further comprising:
a first surface that cultures the stem cells or the progenitor cells in the culture area,
wherein the growth progress is the coverage of the stem cells or the progenitor cells on the first surface

9. The cell culture device according to claim 8, further comprising:
an imaging part that takes images of the stem cells or the progenitor cells,
wherein the controller calculates the coverage based on the images obtained from the imaging part.

10. The cell culture device according to claim 9, further comprising:
a display screen that views the coverage.

11. The cell culture device according to claim 5, wherein the controller controls the oxygen adjusting part to change the first oxygen supply to the second oxygen supply based on the coverage.

12. The cell culture device according to claim 1, further comprising:
an imaging part that takes images of the stem cells or the progenitor cells,
wherein the controller calculates a time-series change in average size of the stem cells or the progenitor cells based on the images obtained from the imaging part.

13. The cell culture device according to claim 11, further comprising:
a display screen that views the time-series change.

14. The cell culture device according to claim 12, wherein the controller, based on the time-series change, identifies the period after the average size of the stem cells or the progenitor cells reaches its maximum level, out of the whole culture period, during which the stem cells or the progenitor cells self-replicate, and controls the oxygen adjusting part to change the first oxygen supply to the second oxygen supply.

15. The cell culture device according to claim 14, wherein the controller controls the oxygen adjusting part to change the first oxygen supply to the second supply in the period, during which the average size of the stem cells or the progenitor cells is constant after the average size reaches its maximum level.
